# EUROPÄISCHE PATENTSCHRIFT

(11) **EP 0 442 087 B1**
(45) Veröffentlichungstag und Bekanntmachung des Hinweises auf die Patenterteilung: **08.12.1993**
(21) Anmeldenummer: 90124183.6
(22) Anmeldetag: 14.12.1990
(51) Int. Cl.: C07C 17/00, C07C 19/08, C07C 23/08

(54) **Verfahren zur Herstellung von gesättigten, fluorhaltigen und chlorfreien Kohlenwasserstoffen**
Process for preparing saturated fluorinated hydrocarbons without chlorine
Procédé pour la fabrication d'hydrocarbures fluorés saturés sans chlore

(30) Priorität: 14.02.1990 DE 4004494
(43) Veröffentlichungstag der Anmeldung: 21.08.1991
(73) Patentinhaber: BAYER AG, 51368 Leverkusen (DE)
(72) Erfinder: Bielefeldt, Dietmar, Dr., W-4030 Ratingen 6 (DE); Braden, Rudolf, Dr., W-5068 Odenthal (DE); Negele, Michael, Dr., W-5000 Köln 80 (DE); Ziemann, Heinz, Dr., W-5653 Leichlingen 1 (DE)

(56) Entgegenhaltungen:
- DE-A- 3 735 467
- DE-A- 3 818 692
- US-A- 4 873 381
- CHEMICAL ABSTRACTS, Band 101, Nr. 7, 13. August 1984, Columbus, Ohio, USA Y. HUANG et al. "Palldiumcatalyzed hydrogenation of polyfluoroalkenes andalkynes" Seite 572, Spalte 1, Zusammenfassung-Nr. 54 508u

## Beschreibung

Die vorliegende Erfindung betrifft ein verbessertes Verfahren zur Herstellung von gesättigten, fluorhaltigen und chlorfreien Kohlenwasserstoffen.

Es ist bekannt, daß man durch katalytische Hydrierung von 2-Chlor- und/oder 2,3-Dichlor-1,1,1,4,4,4-hexafluorbuten bei 50 ± 10°C 1,1,1,4,4,4-Hexafluorbutan erhalten kann (siehe Huang Yaozeng et al., Youji Huoxue (2), S. 125 bis 128 (1984), referiert in C.A. 101, 54 548 u). Das nach der Reaktion vorliegende Gemisch enthält dabei ausgehend von der 2-Chlor-Verbindung 56 %, ausgehend von der 2,3-Dichlor-Verbindung nur 18 % des gewünschten 1,1,1,4,4,4-Hexafluorbutans.

Gesättigte, fluorhaltige und chlorfreie Kohlenwasserstoffe sind neuerdings von besonderem technischen Interesse als die Ozonschicht der Erdatmosphäre nicht negativ beeinflussende Dämm- und Treibgase für Kunststoff-Schaumstoffe und als Arbeitsflüssigkeit für Wärmepumpensysteme (siehe z.B. DE-OS 37 35 467 und DE-OS 38 18 692).

Es wurde nun ein verbessertes Verfahren zur Herstellung von gesättigten, fluorhaltigen und chlorfreien Kohlenwasserstoffen der Formel (I)

R_{f}-CH₂-CH₂-R_{f} (I),

in der
- R_{f}: jeweils für -(-CF₂-)ₙ-CF₃ mit n = 0 bis 7, beide n zusammen jedoch höchstens 10 oder beide R_{f} gemeinsam für -CF₂-CF₂-CF₂- stehen,

gefunden, indem man ungesättigte, fluor- und chlorhaltige Kohlenwasserstoffe der Formel (II)

R_{f}-CX=CCl-R_{f} (II),

in der
- X: für Wasserstoff oder Chlor steht und
- R_{f}: die bei Formel (I) angegebene Bedeutung hat,

katalytisch hydriert, das dadurch gekennzeichnet ist, daß man die Hydrierung in der Gasphase bei Temperaturen über 80°C durchführt.

Vorzugsweise steht in den Formeln (I) und (II) R_{f} für -(-CF₂-)ₙ-CF₃ mit n = 0 bis 2.

Bei den Ausgangsverbindungen der Formel (II) handelt es sich z.B. um 2-Chlor-1,1,1,4,4,4-hexafluorbuten-2, 2,3-Dichlor-1,1,1,4,4,4-hexafluorbuten-2, 1-Chlor-3,3,4,4,5,5-hexafluorcyclopenten und 1,2-Dichlor-3,3,4,4,5,5-hexafluorcyclopenten. Diese Verbindungen sind alle bekannt und gut zugänglich.

Die katalytische Hydrierung kann auf an sich bekannte Weise durchgeführt werden, beispielsweise indem man ein Gemisch aus einer Verbindung der Formel (II), mit Wasserstoff über einen fest angeordneten Hydrierkatalysator leitet. Das Molverhältnis der Verbindung der Formel (II) zu Wasserstoff kann beispielsweise 1:3 bis 1:50 betragen. Vorzugsweise beträgt es 1:4 bis 1:20.

Die Hydrierung kann beispielsweise bei Normaldruck oder bei erhöhten Drucken, beispielsweise im Bereich von Normaldruck bis 20 bar durchgeführt werden. Vorzugsweise wird sie bei Normaldruck durchgeführt.

Als Hydrierkatalysatoren kommen insbesondere solche in Frage, die Übergangsmetalle auf Trägermaterialien enthalten. Von den Übergangsmetallen sind Palladium und Platin bevorzugt, insbesondere Palladium. Beispiele für Trägermaterialien sind Kohle, z.B. in der Form von Kohlefasern, Petrolkoks oder Aktivkohlen, Aluminiumoxide, Siliciumdioxide, Bariumsulfat, Spinelle, Silikate und Titandioxid. Bevorzugt sind Aktivkohlen und Lithium-Aluminium-Spinelle. Die Katalysatoren können beispielsweise 0,5 bis 50 g Übergangsmetall pro Liter Trägermaterial enthalten. Vorzugsweise liegt dieser Gehalt im Bereich 2 bis 20 g/l.

Die Strömungsgeschwindigkeit des Reaktionsgemisches und die Katalysatormenge kann man beispielsweise so wählen, daß sich Katalysatorbelastungen im Bereich von 20 bis 2.000 g/l·h ergeben. Bevorzugt sind Belastungen im Bereich von 50 bis 800 g/l·h.

Es ist ein wesentliches Merkmal der vorliegenden Erfindung, daß man die katalytische Hydrierung bei Temperaturen über 80°C durchführt. Bevorzugt sind Temperaturen im Bereich 90 bis 400°C, insbesondere solche von 95 bis 250°C.

Überraschenderweise werden erfindungsgemäß gesättigte, fluorhaltige und chlorfreie Kohlenwasserstoffe der Formel (I) in deutlich besseren Ausbeuten erhalten als bei dem bekannten, bei niedrigeren Temperaturen durchgeführten Verfahren. Beim erfindungsgemäßen Verfahren liegen die Umsätze und die Selektivitäten im allgemeinen jeweils über 80 %, häufig liegen die Umsätze bei über 90 %. Dies ist insbesondere deshalb überraschend, weil die Selektivität mit steigender Temperatur häufig abnimmt und bei den erfindungsgemäß einzusetzenden Stoffen bei höherer Temperatur zusätzlich die Gefahr der Abspaltung von Fluorwasserstoff und damit die Gefahr der Bildung minderfluorierter Produkte besteht.

Das nach der erfindungsgemäßen Umsetzung vorliegende Gemisch kann beispielsweise aufgearbeitet werden, indem man den gebildeten Chlorwasserstoff gasförmig entweichen läßt und den bei Normaltemperatur hinterbleibenden Rest destilliert. Um besonders reine Produkte der Formel (I) zu erhalten, kann man beispielsweise das nach der Umsetzung vorliegende Gemisch mit einer wässrig-alkalischen Lösung waschen, danach die organische Phase abtrennen und diese über Aktivkohle und Magnesium destillieren.

### Beispiele

Beschreibung der bei der Durchführung der Beispiele verwendeten Apparatur:

Ein senkrecht stehendes, beheizbares Quarzrohr wurde mit 200 ml des jeweils angegebenen Katalysators gefüllt und nach Spülung mit Stickstoff bei Normaldruck mit den jeweils angegebenen Einsätzen beaufschlagt. Nach 25 Minuten wurden die das Quarzrohr verlassenden Gase gaschromatographisch untersucht.

### Beispiel 1

Einsätze: 0,37 Mol/h CF₃-CH=CCl-CF₃, 2,5 Mol/h Wasserstoff.
Katalysator: Palladium auf Li-Al-Spinell (18 g/l).
Reaktionsbedingungen: 150°C, Normaldruck.
Katalysatorbelastung: 360 g/l·h.
Umsatz: 91 %.
Selektivität für CF₃-CH₂-CH₂-CF₃: 78 %.

### Beispiel 2

Einsätze: 0,66 Mol/h CF₃-CH=CCl-CF₃, 3 Mol/h Wasserstoff.
Katalysator: wie in Beispiel 1.
Reaktionsbedingungen: 200°C, Normaldruck.
Katalysatorbelastung: 650 g/l·h.
Umsatz: 86 %.
Selektivität für CF₃-CH₂-CH₂-CF₃: 81 %.

### Beispiel 3

Einsätze: 0:41 Mol/h CF₃-CH=CCl-CF₃, 2,5 Mol/h Wasserstoff.
Katalysator: Palladium auf Aktivkohle (5 g/l).
Reaktionsbedingungen: 100°C: Normaldruck.
Katalysatorbelastung: 410 g/l·h.
Umsatz: 100 %.
Selektivität für CF₃-CH₂-CH₂-CF₃: 100 %.

### Beispiel 4

Einsätze: 0,33 Mol/h CF₃-CH=CCl-CF₃, 2 Mol/h Wasserstoff.
Katalysator: wie bei Beispiel 3.
Reaktionsbedingungen: 200°C, Normaldruck.
Katalysatorbelastung: 320 g/l·h.
Umsatz: 89 %.
Selektivität für CF₃-CH₂-CH₂-CF₃: 91 %.

### Beispiel 5

Einsätze: 0,41 Mol/h CF₃-CH=CCl-CF₃, 2,5 Mol/h Wasserstoff.
Katalysator: Palladium auf Li-Al-Spinell (5 g/l).
Reaktionsbedingungen: 100°C, Normaldruck.
Katalysatorbelastung: 410 g/l·h.
Umsatz: 82 %.
Selektivität für CF₃-CH₂-CH₂-CF₃: 83 %.

### Bespiel 6

Einsätze: 0,37 Mol/h CF₃-CCl=CCl-CF₃, 3,7 Mol/h Wasserstoff.
Katalysator: Palladium auf Li-Al-Spinell (18 g/l).
Reaktionsbedingungen: 150°C, Normaldruck.
Katalysatorbelastung: 440 g/l·h.
Umsatz: 98 %.
Selektivität für CF₃-CH₂-CH₂-CF₃: 81,5 %.

### Beispiel 7

Durchführung wie bei Beispiel 6, jedoch bei 200°C.
Umsatz: 93,9 %.
Selektivität für CF₃-CH₂-CH₂-CF₃: 84,3 %.

### Beispiel 8

Einsätze: 0,17 Mol/h CF₃-CCl=CCl-CF₃, 2,5 Mol/h Wasserstoff.
Katalysator: wie bei Beispiel 6.
Reaktionsbedingungen: 200°C, Normaldruck.
Katalysatorbelastung: 200 g/l·h.
Umsatz: 99,5 %.
Selektivität für CF₃-CH₂-CH₂-CF₃: 93,3 %.

### Beispiel 9

Einsätze: 0,17 Mol/h 1,2-Dichlor-hexafluorcyclopenten, 2,5 Mol/h Wasserstoff.
Katalysator: Palladium auf Li-Al-Spinell (18 g/l).
Reaktionsbedingungen: 200°C, Normaldruck.
Katalysatorbelastung: 210 g/l·h.
Umsatz: 98,5 %.
Selektivität für 1,1,2,2,3,3-Hexafluorcyclopentan: 98 %.

### Beispiel 10

Einsätze: 6,3 Mol 1,2-Dichlor-hexafluorcyclopenten, 45 Mol Wasserstoff im Verlauf von 7,7 Stunden.
Katalysator: wie bei Beispiel 9.
Reaktionsbedingungen: 200°C, Normaldruck.
Katalysatorbelastung: 200 g/l·h.
Ausbeute: 1.035 g
Selektivität für 1,1,2,2,3,3-Hexafluorcyclopentan: 97 %.

## Patentansprüche

1. Verfahren zur Herstellung von gesättigten, fluorhaltigen und chlorfreien Kohlenwasserstoffen der Formel (I)
R_{f}-CH₂-CH₂-R_{f} (I),
in der
R_{f} jeweils für -(-CF₂-)ₙ-CF₃ mit n = 0 bis 7, beide n zusammen jedoch höchstens 10 oder beide R_{f} gemeinsam für -CF₂-CF₂-CF₂- stehen,
indem man ungesättigte, fluor- und chlorhaltige Kohlenwasserstoffe der Formel (II)
R_{f}-CX=CCl-R_{f} (II),
in der
X für Wasserstoff oder Chlor steht und
R_{f} die bei Formel (I) angegebene Bedeutung hat,
katalytisch hydriert, dadurch gekennzeichnet, daß man die Hydrierung in der Gasphase bei Temperaturen über 80°C durchführt.

2. Verfahren nach Anspruch 1, dadurch gekennzeichnet, daß das Molverhältnis der Verbindung der Formel (II) zu Wasserstoff 1:3 bis 1:50 beträgt.

3. Verfahren nach Ansprüchen 1 bis 2, dadurch gekennzeichnet, daß man es bei Drucken im Bereich von Normaldruck bis 20 bar durchführt.

4. Verfahren nach Ansprüchen 1 bis 3, dadurch gekennzeichnet, daß man als Katalysator Übergangsmetalle auf Trägermaterialien einsetzt.

5. Verfahren nach Ansprüchen 1 bis 4, dadurch gekennzeichnet, daß man als Katalysatoren Palladium oder Platin auf Kohle, Aluminiumoxid, Siliciumdioxid, Bariumsulfat, Spinellen, Silikaten oder Titandioxid einsetzt.

6. Verfahren nach Ansprüchen 1 bis 5, dadurch gekennzeichnet, daß man die Strömungsgeschwindigkeit des Reaktionsgemisches und die Katalysatormenge so wählt, daß sich Katalysatorbelastungen im Bereich von 20 bis 2.000 g/l·h ergeben.

7. Verfahren nach Ansprüchen 1 bis 6, dadurch gekennzeichnet, daß man es bei Temperaturen im Bereich 90 bis 400°C durchführt.

8. Verfahren nach Ansprüchen 1 bis 7, dadurch gekennzeichnet, daß man es bei Temperaturen im Bereich von 95 bis 250°C durchführt.

## Claims

1. Process for the preparation of saturated, fluorine- containing and chlorine-free hydrocarbons of the formula (I)
R_{f}-CH₂-CH₂-R_{f} (I)
in which
R_{f} in each case represents -(-CF₂-)ₙ-CF₃, where n = 0 to 7, both n together, however, represent at most 10 or both R_{f} jointly represent -CF₂-CF₂-CF₂-,
by catalytically hydrogenating unsaturated, fluorine- and chlorine-containing hydrocarbons of the formula (II)
R_{f}-CX=CCl-R_{f} (II)
in which
X represents hydrogen or chlorine and
R_{f} has the meaning indicated in formula (I),
characterized in that the hydrogenation is carried out in the gas phase at temperatures above 80°C.

2. Process according to Claim 1, characterized in that the molar ratio of the compound of the formula (II) to hydrogen is 1:3 to 1:50.

3. Process according to Claims 1 to 2, characterized in that it is carried out at pressures in the range from normal pressure up to 20 bar.

4. Process according to Claims 1 to 3, characterized in that transition metals on support materials are employed as the catalyst.

5. Process according to Claims 1 to 4, characterized in that palladium or platinum on carbon, alumina, silica, barium sulphate, spinels, silicates or titanium dioxide are employed as catalysts.

6. Process according to Claims 1 to 5, characterized in that the flow rate of the reaction mixture and the catalyst quantity are chosen such that catalyst weight hourly space velocities in the range from 20 to 2,000 g/l·h result.

7. Process according to Claims 1 to 6, characterized in that it is carried out at temperatures in the range 90 to 400°C.

8. Process according to Claims 1 to 7, characterized in that it is carried out at temperatures in the range from 95 to 250°C.

## Revendications

1. Procédé pour la préparation d'hydrocarbures saturés fluorés mais exempts de chlore, de formule (I):
R_{f}-CH₂-CH₂-R_{f} (I),
dans laquelle
chacun des symboles R_{f} représente -(-CF₂-)ₙ-CF₃ avec n=0 à 7, la somme des deux indices n étant toutefois de 10 au maximum, ou bien les deux symboles Rf représentent ensemble -CF₂-CF₂-CF₂-,
par hydrogénation catalytique d'hydrocarbures insaturés fluorés et chlorés de formule (II):
R_{f}-CX=CCl-R_{f} (II),
danslaquelle
X représente l'hydrogène ou le chlore et
R_{f} a les significations indiquées en référence à la formule (I),
caractérisé en ce que l'on hydrogène en phase gazeuse à des températures supérieures à 80°C.

2. Procédé selon la revendication 1, caractérisé en ce que le rapport molaire entre le composé de formule (II) et l'hydrogène va de 1:3 à 1:50.

3. Procédé selon les revendications 1 et 2, caractérisé en ce que l'on opère à des pressions dans l'intervalle allant de la pression normale à 20 bar.

4. Procédé selon les revendications 1 à 3, caractérisé en ce que l'on utilise en tant que catalyseurs des métaux de transition sur des matières de support.

5. Procédé selon les revendications 1 à 4, caractérisé en ce que l'on utilise en tant que catalyseurs le palladium ou le platine sur charbon, alumine, silice, sulfate de baryum, spinelle, silicate ou dioxyde de titane.

6. Procédé selon les revendications 1 à 5, caractérisé en ce que l'on choisit la vitesse d'écoulement du mélange de réaction et la quantité de catalyseur de manière à parvenir à des charges de catalyseur dans l'intervalle de 20 à 2 000 g/l.h.

7. Procédé selon les revendications 1 à 6, caractérisé en ce que l'on opère à des températures dans l'intervalle de 90 à 400°C.

8. Procédé selon les revendications 1 à 7, caractérisé en ce que l'on opère à des températures dans l'intervalle de 95 à 250°C.
